# EUROPÄISCHE PATENTSCHRIFT

(11) **EP 0 709 368 B1**
(45) Veröffentlichungstag und Bekanntmachung des Hinweises auf die Patenterteilung: **31.03.1999**
(21) Anmeldenummer: 95116098.5
(22) Anmeldetag: 12.10.1995
(51) Int. Cl.: C07C 233/15, A01N 37/22

(54) **Herbizide Mittel auf Basis von N-(4-Fluor-phenyl)-N-isopropyl-chloracetamid**
Herbicidal agent based on the N-(4-fluorphenyl)-N-isopropyl-chloroacetamide
Agent herbicide à base du N-(4-fluoro-phényl)-N-isopropyl chloroacétamide

(30) Priorität: 25.10.1994 DE 4438001
(43) Veröffentlichungstag der Anmeldung: 01.05.1996
(60) Teilanmeldung: 98115670.6
(73) Patentinhaber: BAYER AG, 51368 Leverkusen (DE)
(72) Erfinder: Lantzsch, Reinhard, Dr., D-42115 Wuppertal (DE); Förster, Heinz, Dr., D-56337 Kadenbach (DE); Schmidt, Thomas, Dr., D-42781 Haan (DE); Steinbeck, Karl, Dr., D-51399 Burscheid (DE); Dollinger, Markus, Dr., D-51381 Leverkusen (DE); Santel, Hans-Joachim, Dr., D-51371 Leverkusen (DE)

(56) Entgegenhaltungen:
- BE-A- 736 779
- DE-A- 2 027 822
- FR-A- 2 318 861
- US-A- 4 418 021
- US-E- R E26 961
- PATENT ABSTRACTS OF JAPAN vol. 12 no. 365 (C-532) [3212] ,29.September 1988 & JP-A-63 119447 (HOKKO CHEM IND CO LTD) 24.Mai 1988,
- EUGEN MÜLLER 'Methoden der organischen chemie, Band XI/2' 1958 , GEORG THIEME VERLAG , STUTTGART * Seite 13, letzter Absatz *

## Beschreibung

Die Erfindung betrifft die Verwendung der bekannten Verbindung N-(4-Fluorphenyl)-N-isopropyl-chloracetamid als Wirkstoff in selektiv-herbiziden Mitteln.

Es ist bekannt, daß bestimmte substituierte Chloressigsäureanilide als Herbizide verwendet werden können. So beschreibt Dokument BE-A-736779 die Verwendung von gegebenenfalls kernfluorierten N-(C₅-Alkinyl)-chloracetaniliden, wie z.B. N-(4-Fluor-phenyl)-N-(3-methyl-1-butin-3-yl)-chloracetamid, als Herbizide.

Ferner wird in Dokument US-E-RE26961 die Verwendung von gegebenenfalls kernhalogeniertem N-Methyl-, N-Ethyl-, N-Butyl- und N-(2-Chlorallyl)-chloracetanilid als Herbizide beschrieben. Das Dokument DE-A-2027822 beschreibt ein Verfahren zur Herstellung von herbizid wirksamen, gegebenenfalls kernchlorierten N-Alkyl-chloracetaniliden, z.B. von N-(4-Chlor-phenyl)-N-methylchloracetamid. Im Dokument JP-A-63119447 werden kernfluorierte, gegebenenfalls (z.B. durch Alkylreste) N-substituierte Chloracetanilide, wie z.B. N-(2-Methyl-3-fluor-6-ethyl-phenyl)-chloracetamid, und deren Verwendung als Herbizide beschrieben. Des weiteren offenbart das Dokument FR-A-2318861 die Verwendung von N-(2,3-Dimethyl-phenyl)-N-isopropyl-chloracetamid als Herbizid.

N-(4-Fluor-phenyl)-N-isopropyl-chloracetamid ist bereits als Zwischenprodukt für Agrochemikalien bekannt geworden (vgl. DE-A-2633159, US-A-4140774, US-A-4418021). Die Verwendung der genannten Verbindung als Herbizid ist jedoch bisher nicht bekannt geworden.

Es wurde nun gefunden, daß die Verbindung N-(4-Fluor-phenyl)-N-isopropylchloracetamid der nachstehenden Formel (I) 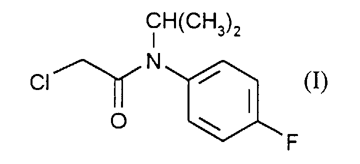 sehr gut zur selektiven Bekämpfung von Unkräutern in wichtigen Kulturen verwendet werden kann.

Ein Verfahren zur Herstellung der Verbindung der Formel (I) durch Umsetzung von N-Isopropyl-4-fluor-anilin mit Chloracetylchlorid in Gegenwart von Pyridin ist bekannt (vgl. US-A-4453965).

Die Verbindung der Formel (I) kann in Defoliants, Desiccants, Krautabtötungsmitteln und insbesondere in Unkrautvernichtungsmitteln verwendet werden. Unter Unkraut im weitesten Sinne sind alle Pflanzen zu verstehen, die an Orten aufwachsen, wo sie unerwünscht sind.

Die Verbindung der Formel (I) kann z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der Verbindung der Formel (I) ist jedoch keineswegs auf diese Gattungen beschränkt, sondern erstreckt sich in gleicher Weise auch auf andere Pflanzen.

Die Verbindung der Formel (I) eignet sich in Abhängigkeit von der Konzentration zur Totalunkrautbekämpfung z.B. auf Industrie- und Gleisanlagen und auf Wegen und Plätzen mit und ohne Baumbewuchs. Ebenso kann die Verbindung zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäße Verbindung der Formel (I) eignet sich insbesondere zur selektiven Bekämpfung von monokotylen und dikotylen Unkräutern in monokotylen Kulturen, wie z.B. in Mais, vor allem im Vorauflauf-Verfahren.

Die Verbindung der Formel (I) kann in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen des Wirkstoffs mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäße Verbindung der Formel (I) kann als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Verbindung der Formel (I) kann als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die Verbindung der Formel (I) kann sowohl vor als auch nach dem Auflaufen der Pflanzen appliziert werden. Sie kann auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Verwendung der Verbindung der Formel (I) geht aus den nachfolgenden Beispielen hervor.

Anwendungsbeispiel:

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.

Es bedeuten:
- 0% =: keine Wirkung (wie unbehandelte Kontrolle)
- 100 % =: totale Vernichtung

In diesem Test zeigt die Verbindung der Formel (I) bei sehr guter Verträglichkeit gegenüber Kulturpflanzen, wie z.B. Mais, starke Wirkung gegen Unkräuter (vgl. Tabelle A).

**Tabelle A:**

| Pre-emergence-Test/Gewächshaus | | | | | | |
|---|---|---|---|---|---|---|
| Aufwandmenge | Mais | Cynodon | Echinochloa | Setaria | Amaranthus | Galinsoga |
| 1000 g/ha (1) | 0 | 100 | 100 | 100 | 90 | 95 |

## Patentansprüche

1. Verwendung von N-(4-Fluor-phenyl)-N-isopropyl-chloracetamid der Formel (I) 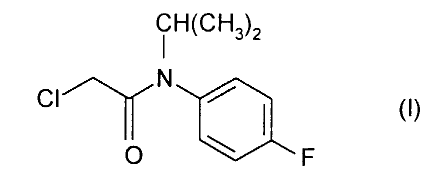 zur Bekämpfung von Unkraut.

2. Verfahren zur Bekämpfung von Unkraut, dadurch gekennzeichnet, daß man N-(4-Fluor-phenyl)-N-isopropyl-chloracetamid (I) auf die Unkräuter oder ihren Lebensraum einwiken läßt.

3. Verfahren zur Herstellung von herbiziden Mitteln, dadurch gekennzeichnet, daß man N-(4-Fluor-phenyl)-N-isopropyl-chloracetamid (I) mit Streckmitteln und/oder oberflächenaktiven Mitteln vermischt.

## Claims

1. Use of N-(4-fluoro-phenyl)-N-isopropyl-chloroacetamide, of the formula (I) 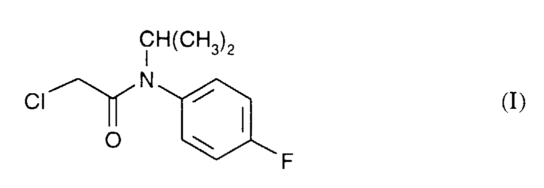 for combating weed.

2. Method of combating weed, characterized in that N-(4-fluoro-phenyl)-N-isopropylchloroacetamide (I) is allowed to act on the weeds or their environment.

3. Process for the preparation of herbicidal compositions, characterized in that N-(4-fluoro-phenyl)-N-isopropyl-chloroacetamide (I) is mixed with extenders and/or surface-active agents.

## Revendications

1. Utilisation du N-(4-fluoro-phényl)-N-isopropylchloracétamide répondant à la formule (I) 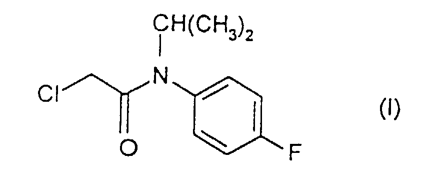 pour lutter contre les mauvaises herbes.

2. Procédé pour lutter contre les mauvaises herbes, caractérisé en ce qu'on laisse agir le N-(4-fluorophényl)-N-isopropyl-chloracétamide (I) sur les mauvaises herbes ou sur leur biotope.

3. Procédé pour la préparation d'agents herbicides, caractérisé en ce qu'on mélange le N-(4-fluorophényl)-N-isopropyl-chloracétamide (I) avec des diluants et/ou des agents tensioactifs.
